# EUROPÄISCHE PATENTSCHRIFT

(11) **EP 1 536 756 B1**
(45) Veröffentlichungstag und Bekanntmachung des Hinweises auf die Patenterteilung: **24.02.2016**
(21) Anmeldenummer: 03794851.0
(22) Anmeldetag: 08.08.2003
(51) Int. Cl.: A61K 8/41, A61K 8/49, A61Q 5/02, A61Q 5/00

(54) **WIRKSTOFFKOMBINATIONEN AUS CLIMBAZOL, PIROCTON OLAMIN UND UNDECYLENAMIDOPROPYLBETAIN. VERWENDUNG ZUR BEKÄMPFUNG VON KOPFSCHUPPEN**
COMBINATIONS OF ACTIVE SUBSTANCES, COMPRISING CLIMBAZOLE, PIROCTON OLAMIN, AND UNDECYLENAMIDOPROPYL BETAINE, AND USE THEREOF FOR CONTROLLING DANDRUFF
COMBINAISON DE SUBSTANCES ACTIVES COMPRENANT DU CLIMBAZOLE, DU PIROCTON OLAMINE ET DE L'UNDECYLENE AMIDOPROPYLE BETAINE. SON UTILISATION POUR LA LUTTE CONTRE LES PELLICULES

(30) Priorität: 27.08.2002 DE 10240029
(43) Veröffentlichungstag der Anmeldung: 08.06.2005
(73) Patentinhaber: Beiersdorf AG, 20245 Hamburg (DE)
(72) Erfinder: ARGEMBEAUX, Horst, 21465 Wentorf (DE); BLUCK, Manuela, 21031 Hamburg (DE)
(86) Internationale Anmeldenummer: PCT/EP2003/008809
(87) Internationale Veröffentlichungsnummer: WO 2004/024107

(56) Entgegenhaltungen:
- EP-A- 1 050 298
- FR-A- 2 774 900
- FR-A- 2 792 195

## Beschreibung

Die vorliegende Erfindung betrifft kosmetische Wirkstoffkombinationen und Zubereitungen, solche Wirkstoffkombinationen enthaltend.

Insbesondere betrifft die vorliegende Erfindung haarkosmetische Reinigungszubereitungen oder haarkosmetische Reinigungsmittel mit einem Gehalt an Substanzen, die die Kopfhaut derartig beeinflussen, daß lose Hautschuppen entfernt und die erneute Bildung sichtbarer Schuppen verhindert wird. In einer bevorzugten Ausführungsform betrifft die vorliegende Erfindung Wirkstoffkombinationen und Zubereitungen damit, die dazu dienen, das Haar und die Kopfhaut zu pflegen.

Der Mechanismus der übermäßigen Bildung von Kopfschuppen ist noch nicht vollständig erforscht, nach Bonadeo und Lüpke sind jedoch Mikroben daran beteiligt. In Kombination mit Lipasen, Peroxidasen, Licht und Luft werden aus dem Sebum, das von den Talgdrüsen der Kopfhaut abgesondert wird, freie Fettsäuren und Lipoperoxide gebildet. Diese üben eine chronische Reizwirkung aus, die zu Mitosebeschleunigung in der Keimschicht und somit zu Parakeratose, der grundlegenden Voraussetzung für Kopfschuppen, führt.

Wird dieser Mechanismus durch Abtöten der Mikroben durchbrochen, fehlt die Vorraussetzung zur Bildung von Kopfschuppen. Mit Hilfe antmikrobiell wirkender Substanzen können somit wirksame Antischuppenmittel formuliert werden. Solche Präparate nach dem aktuellen Stand der Technik haben jedoch nur eine mäßige Wirkung, die nach Absetzen der Behandlung sofort wieder nachläßt.

Einfache Schuppenbildung (Pityriasis simplex): "herkömmliche" Schuppen und eine leichte Hautreizung, die mit einem speziellen Pflegeprodukt gut behandelt werden können.

Stärkere Schuppung (klassische Form der seborrhoischen Dermatitis): Diese Form kann sich auch auf andere Hautpartien ausdehnen. Wenn dies der Fall ist, ist in der Regel medizinische Hilfe vonnöten.

Wenn Schuppen auftreten, ist das kein Zeichen mangelnder Körperpflege. Die Ursache ist vielmehr ein Hefepilz mit Namen Pityrosporum ovale oder Malassezia furfur, der bei fast allen Menschen vorkommt. Er siedelt sich vorzugsweise in Hautregionen mit einer erhöhten Talgproduktion an. Im Haarschopf findet er seine optimalen Vermehrungsbedingungen: die Sonne kann nicht bis auf die Haut dringen und die Luftzirkulation ist stark eingeschränkt. Es herrscht ein feuchtwarmes Klima. Schuppen sind also kein Haar-, sondern ein Hautproblem.

Der Hefepilz sorgt dafür, daß vermehrt Hautpartikel abgestoßen werden und sich die Zusammensetzung des Hautfettes verändert. Dadurch verklumpen die Hautpartikel und werden zu dem, was man als "Schuppen" bezeichnet

Die Entstehung des Schuppenproblems hängt allerdings auch von diversen anderen Faktoren ab. Sowohl innere Faktoren, wie beispielsweise genetische Veranlagung und Streß, als auch äußere, wie falsche Ernährung und falsche Haarpflege spielen eine entscheidende Rolle.

Die vorliegende Erfindung betrifft antimycotisch wirksame und gegen den Befall mit Mycobionten geschützte Zubereitungen, bevorzugt kosmetischen oder dermatologischen Zubereitungen.

Pilze, auch Fungi [fungus = lat. Pilz], Mycota [µυκησ = grch. Pilz] oder Mycobionten genannt, zählen zu den Eucaryonten. Eucaryonten sind Lebewesen, deren Zellen (Eucyten) im Gegensatz zu denen der sogenannten Procaryonten (Procyten) über einen durch Kemhülle und Kernmembran vom restlichen Cytoplasma abgegrenzten Zellkern verfügen. Der Zellkern enthält die Erbinformation in Chromosomen gespeichert.

Zu Vertretern der Mycobionten zählen beispielsweise Hefen (Protoascomycetes), Schimmelpilze (Plectomycetes), Mehltau (Pyrenomycetes), der falsche Mehltau (Phycomycetes) und die Ständerpilze (Basidiomycetes).

Pilze, auch nicht die Basidiomyceten, sind keine pflanzlichen Organismen, haben aber wie diese eine Zellwand, zellsaftgefüllte Vakuolen und eine mikroskopisch gut sichtbare Plasmaströmung. Sie enthalten keine photosynthetischen Pigmente und sind C-heterotroph. Sie wachsen unter aeroben Bedingungen und gewinnen Energie durch Oxidation organischer Substanzen. Einige Vertreter, beispielsweise Hefen, sind allerdings fakultative Anaerobier und zur Energiegewinnung durch Gärungsprozesse befähigt.

Dermatomycosen sind Krankheiten, bei der gewisse Pilzarten, insbesondere Dermatophyten, in die Haut und Haarfollikel eindringen. Die Symptome von Dermatomycosen sind beispielsweise Bläschen, Exfoliation, Rhagaden und Erosion, meist verbunden mit Juckreiz oder allergischem Ekzem.

Dermatomycosen können im wesentlichen in folgende vier Gruppen unterteilt werden: Dermatophytien (z.B. Epidermophytie, Favus, Mikrosporie, Trichophytie), Hefemycosen (z.B. Pityriasis und andere Pityosporum-bedingte Mycosen, Candida-Infektionen, Blastomycose, Busse-Buschke-Krankheit, Torulose, Piedra alba, Torulopsidose, Trichosporose), Schimmelmycosen (z.B. Aspergillose, Kephalosporidose, Phycomycose und Skopulariopsidose), Systemmycosen (z.B. Chromomycose, Coccidiomycose, Histoplasmose).

Zu den pathogenen und fakultativ pathogenen Keimen gehören aus der Gruppe der Hefen Candida-Arten (z.B. Candida albicans) und solche der Familie Pityrosporum. Pityrosporum-Arten, insbesondere Pityrosporum ovale, sind für Hauterkrankungen wie Pityriasis versicolor, Seborrhoe in den Formen Seborrhoea oleosa und Seborrhoea sicca, welche sich vor allem als Seborrhoea capitis (= Kopfschuppen) äußern, seborrhoisches Ekzem und Pityrosporum-Follikulitis verantwortlich zu machen.

Alle Bereiche der menschlichen Haut können von Dermatomycosen befallen werden. Dermatophytien befallen fast ausschließlich Haut, Haare und Nägel. Hefemycosen können auch Schleimhäute und innere Organe befallen, Systemmycosen erstrecken sich regelmäßig auf ganze Organsysteme.

Besonders häufig sind die Körperbereiche betroffen, auf welchen sich durch Kleidung, Schmuck oder Schuhwerk Feuchtigkeit und Wärme stauen können. So gehört der Fußpilz zu den bekanntesten und am weitesten verbreiteten Dermatomycosen. Besonders unangenehm sind weiterhin Pilzerkrankungen der Finger- und Fußnägelbereiche.

Ferner sind Superinfektionen der Haut durch Pilze nicht selten.

Die gesunde menschliche Haut ist mit einer Vielzahl nichtpathogener Mikroorganismen besiedelt. Diese sogenannte Mikroflora der Haut ist nicht nur unschädlich, sie stellt einen wichtigen Schutz zur Abwehr opportunistischer oder pathogener Keime dar.

Bei bestehendem Primärinfekt, d.h., der normalen Keimbesiedelung der Haut, eintretende Neuinfektion mit hohen Keimzahlen eines oder mehrerer oft physiologischer Erreger, beispielsweise Staphylokokken, oft aber auch unphysiologischer Erreger, beispielsweise Candida albicans, kann bei Zusammentreffen ungünstiger Einflüssen eine "Superinfektion" der befallenen Haut auftreten. Die normale Mikroflora der Haut (oder eines anderen Körperorgans) wird dabei von dem Sekundärerreger regelrecht überwuchert.

Solche Superinfektionen können sich, in Abhängigkeit vom betreffenden Keim, in günstig verlaufenden Fällen in unangenehmen Hauterscheinungen (Juckreiz, unschönes äußeres Erscheinungsbild) äußern. In ungünstig verlaufenden Fällen können sie aber zu großflächiger Zerstörung der Haut führen, im schlimmsten Falle sogar im Tode des Patienten gipfeln.

Superinfektionen der vorab geschilderten Art sind z.B. beim Vollbild von AIDS häufig auftretende Sekundärerkrankungen. An sich - jedenfalls in geringen Keimdichten -unschädliche, aber unter Umständen auch ausgesprochen pathogene Keime überwuchern auf diese Weise die gesunde Hautflora. Bei AIDS allerdings sind auch andere Körperorgane von Superinfektionen betroffen.

Ebenso werden derartige Superinfektionen bei einer Vielzahl dermatologischer Erkrankungen, z.B. atopischem Ekzem, Neurodermitis, Akne, seborrhoischer Dermatitis oder Psoriasis beobachtet. Auch viele medizinische und therapeutische Maßnahmen, z.B die Radio- oder Chemotherapie von Tumorerkrankungen, als Nebenwirkung hervorgerufene, medikamentös induzierte Immunsuppression oder aber systemische Antibiotikabehandlung, ebenso wie externe chemische oder physikalische Einflüsse (z.B. Umweltverschmutzung, Smog), fördern das Auftreten von Superinfektionen der äußeren und inneren Organe, insbesondere der Haut und der Schleimhäute.

Zwar ist es im Einzelfalle ohne weiteres möglich, Superinfektionen mit Antibiotika zu bekämpfen. Meistens haben solche Substanzen aber den Nachteil unangenehmer Nebenwirkungen. Oft sind Patienten beispielsweise gegen Penicilline allergisch, weswegen eine entsprechende Behandlung sich in einem solchen Falle verbieten würde.

Ferner haben topisch verabreichte Antibiotika den Nachteil, daß sie die Hautflora nicht nur vom Sekundärerreger befreien, sondern auch die an sich physiologische Hautflora stark beeinträchtigen und der natürliche Heilungsprozeß auf diese Weise wieder gebremst wird.

Eine Aufgabe der vorliegenden Erfindung war, die Nachteile des Standes der Technik zu beseitigen und Substanzen und Zubereitungen, solche Substanzen enthaltend, zur Verfügung zu stellen, durch deren Verwendung Superinfektionen geheilt werden können, wobei die physiologische Hautflora keine nenneswerte Einbußen erleidet.

Darüberhinaus war es eine Aufgabe der vorliegenden Erfindung, Substanzen und kosmetische bzw. dermatologische Zubereitungen, solche Substanzen enthaltend, zur Verfügung zu stellen, die prophylaktisch gegen Superinfektionen wirken.

Schließlich können durch Mycobiontenbefall Zubereitungen, insbesondere Nahrungsmittel, aber auch Kosmetika und dergleichen zerstört werden. Der Schutz vor Infektion erstreckt sich daher auch auf die Produkte selbst.

Behandelt werden Dermatomycosen medikamentös oder mit anderen Methoden, beispielsweise mit Lichtbestrahlung. Gängige Medikamente enthalten Salicylsäure, Kresol, 1-Menthol und andere, die alle äußerlich angewandt werden und regelmäßig gute Heilerfolge zeitigen.

Dennoch haben die bisher bekannten antimycotisch wirksamen Mittel den Nachteil daß sie unangenehm riechen und/oder die beeinträchtigte Hautpartie zusätzlich reizen. In schweren Fällen von Dermatomycosen kann durch das Medikament sogar Schmerz ausgelöst werden.

Eine weitere Aufgabe der vorliegenden Erfindung war also, antimycotisch wirksame Mittel zur Verfügung zu stellen, welche die Nachteile des. Standes der Technik nicht aufweisen. Insbesondere sollten Wirkstoffe zugängig gemacht werden, die folgende Bedingungen erfüllen: .
1) Die biologischen Vorgänge der Haut dürfen nicht beeinträchtigt werden.
2) Die Wirkstoffe sollen keinen ausgeprägten Eigengeruch besitzen.
3) Sie sollen bei Überdosierung oder sonstiger nicht bestimmungsgemäßer Anwendung unschädlich sein.
4) Sie sollen sich nach wiederholter Anwendung nicht auf der Haut anreichern.
5) Pathogene und fakultativ pathogene Mycobionten sollen wirksam bekämpft, die physiologische Mikroflora der Haut im übrigen aber geschont werden.
6) Die wirksamen Prinzipien sollen sich gut in übliche kosmetische oder dermatologische Formulierungen einarbeiten lassen.
7) Die Wirkstoffe sollen die Haut nicht reizen.
8) Zubereitungen, diese Wirkstoffe enthaltend, sollen ebenfalls vor Befall mit Mycobionten geschützt sein.

Es wurde gefunden; und darin liegt die Lösung all dieser Aufgaben, daß Wirkstoffkombinationen, umfassend
a) Climbazol
b) Pirocton olamin
c) Undecylenamidopropylbetain
bzw. die Verwendung solcher Wirkstoffkombinationen als antimycotisches Wirkprinzip in kosmetischen oder dermatologischen Zubereitungen, insbesondere als Wirkprinzip gegen Kopfschuppen, den Nachteilen des Standes der Technik abhelfen.

Climbazol (chemische Bezeichnung: (*RS*)-1-(4-chlorophenoxy)-1-imidazol-1-yl-3,3-dimethyl-butan-2-on, CAS-Nr. 38083-17-9) ist durch folgende chemische Strukturformel gekennzeichnet:

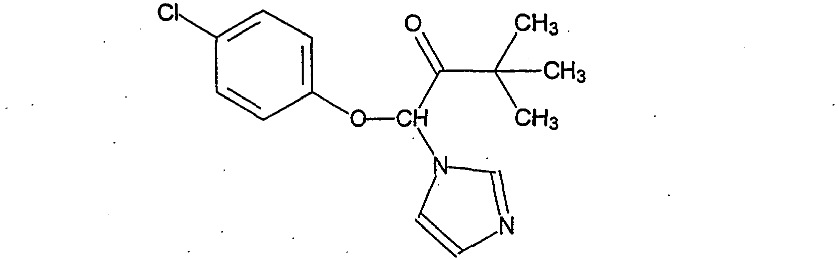

Pirocton olamin (chemische Bezeichnung: (Carboxymethy)-dimethyl-(3-(1-oxoundecylenyl)-amino)-propyl)-ammoniumhydroxid, CAS-Nr. 68890-66-4) ist durch folgende chemische Strukturformel gekennzeichnet:

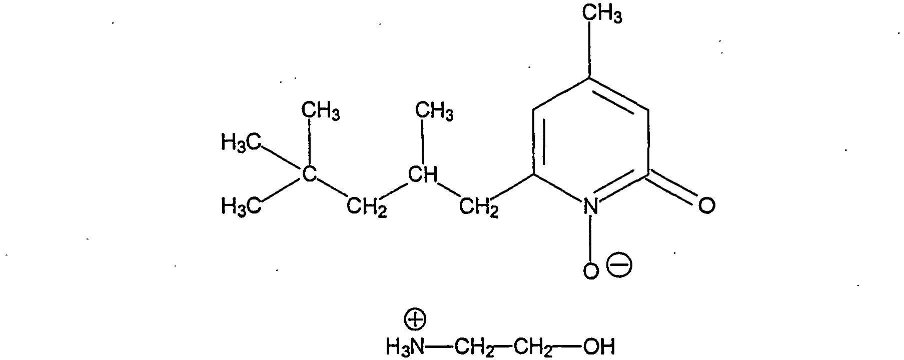

Undecylenamidopropylbetain ist durch folgende chemische Strukturformel gekennzeichnet:

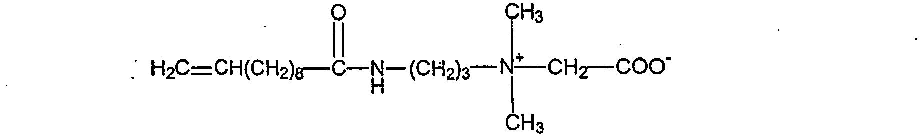

Es ist erfindungsgemäß vorteilhaft, die Gewichtsverhältnisse von Climbazol zu Pirocton olamin zu Undecylenamidopropylbetain wie a : b : c zu wählen, wobei a, b und c unabhängig voneinander rationale Zahlen von 1 bis 10, bevorzugt von 1 bis 8 darstellen können. Insbesondere bevorzugt ist ein Gewichtsverhältnis von etwa 2 : 1 : 8.

Vorteilhaft werden die erfindungsgemäßen Wirkstoffkombinationen in kosmetischen oder dermatologischen Zubereitungen eingesetzt.

Erfindungsgemäße Zubereitungen enthalten vorteilhaft 0,001 bis 5,0 Gew.-% Climbazol, bevorzugt 0,01 bis 2,0 Gew.-%, bezogen auf das Gesamtgewicht der Zubereitungen.

Erfindungsgemäße Zubereitungen enthalten vorteilhaft 0,001 bis 5,0 Gew.-% Pirocton olamin, bevorzugt 0,01 bis 2,0 Gew.-%, jeweils bezogen auf das Gesamtgewicht der Zubereitungen.

Vorteilhaft enthalten erfindungsgemäße Zubereitungen 0,01 bis 25 Gew.-% Undecylenamidopropylbetain, bevorzugt 0,02 bis 5 Gew.-%, jeweils bezogen auf das Gesamtgewicht der Zubereitungen.

Es hat sich in erstaunlicher Weise herausgestellt, daß die erfindungsgemäßen Wirkstoffkombinationen das Wachstum von Mycobionten verhindern, und dies in synergistischer Weise, also überadditiv in bezug auf die Einzelkomponenten.

Die erfindungsgemäßen Wirkstoffkombinationen sind ausgezeichnet wirksam gegen Mycobionten, insbesondere in deren Auswirkungsform der Dermatomycosen. Dabei sind die erfindungsgemäßen Wirkstoffkombinationen insbesondere befähigt, das Wachstum von Hefen, insbesondere der Pityrosporum-Arten, namentlich Pityrosporum ovale, zu verhindern.

Es hat sich ferner in überraschender Weise herausgestellt, daß die erfindungsgemäßen Wirkstoffkombinationen die Bildung von seborrhoischen Erscheinungen, insbesondere Kopfschuppen, verhindern sowie bereits vorhandene seborrhoische Erscheinungen, insbesondere Kopfschuppen, zu beseitigen.

Erfindungsgemäß ist somit auch ein Verfahren zur Bekämpfung seborrhoischer Erscheinungen, insbesondere Kopfschuppen, sowie die Prophylaxe seborrhoischer Erscheinungen, insbesondere Kopfschuppen.

Schließlich hat sich herausgestellt, daß die erfindungsgemäßen Wirkstoffkombinationen den Verderb organischer Substanz, insbesondere kosmetischer und dermatologischer Zubereitungen, durch den Befall mit Mycobionten verhindern können, wenn sie diesen Zubereitungen zugesetzt werden.

Erfindungsgemäß sind somit auch ein Verfahren zur Bekämpfung von Mycobionten, dadurch gekennzeichnet, daß die erfindungsgemäßen Wirkstoffkombinationen, gegebenenfalls in einem geeigneten kosmetischen oder dermatologischen Träger, mit dem durch Mycobionten kontaminierten Bereich in Kontakt gebracht werden, sowie ein Verfahren zum Schutze organischer Produkte vor dem Befall mit Mycobionten, dadurch gekennzeichnet, daß diesen organischen Produkten erfindungsgemäße Wirkstoffkombinationen in wirksamer Menge zugegeben werden.

Ferner war erstaunlich, daß die erfindungsgemäßen Wirkstoffkombinationen besonders gut wirksam sind gegen den für das Entstehen von Kopfschuppen verantwortlichen Keim Pityrosporum ovale und verwandte Keime. Eine bevorzugte Ausführungsform der vorliegenden Erfindung sind mithin gegen Kopfschuppen anzuwendende Formulierungen, beispielsweise Antischuppenshampoos.

Erfindungsgemäß werden die Wirkstoffkombinationen bevorzugt in kosmetischen oder dermatologischen Zusammensetzungen eingesetzt in einem Gehalt von 0,005 - 50,0 Gew.-%, insbesondere 0,01 - 20,0 Gew.-%, bezogen auf das Gesamtgewicht der Zusammensetzung bevorzugt sind. Vorteilhaft enthalten die Zusammensetzungen 0,02 - 10,0 Gew.-%, besonders bevorzugt 0,02 - 8,0 Gew.-% an den erfindungsgemäßen Wirkstoffkombinationen, ganz besonders vorteilhaft 0,5 - 6,0 Gew.-%., jeweils bezogen auf das Gesamtgewicht der Zusammensetzung.

Die erfindungsgemäßen Wirkstoffkombinationen lassen sich ohne Schwierigkeiten in gängige kosmetische oder dermatologische Formulierungen einarbeiten, vorteilhaft in Pumpsprays, Aerosolsprays, Cremes, Salben, Tinkturen, Lotionen, Nagelpflegeprodukte (z.B. Nagellacke, Nagellackentferner, Nagelbalsame) und dergleichen.

Ganz besonders vorteilhaft liegen die erfindungsgemäßen Wirkstoffkombinationen in Form von Antischuppen-Shampoos vor.

Besonders geeignet sind derartige Wirkstoffkombinationen, wenn ihnen als weitere Komponente Alkylpolyglykolether der allgemenen Formel:

CH3-(CH2)ₓ-(CH-O-CH)_{y}-OH

X=9-15;y=6-12

zugesetzt werden.

Bevorzugt enthalten die erfindungsgemäßen Zubereitungen 0,1 - 10,0 Gew.-% eines Alkylpolyglykolethers. Besonders bevorzugt ist Laureth-9 mit 0,5 - 7,0 Gew.-% jeweils bezogen auf das Gesamtgewicht der Zubereitung.

Es ist auch möglich und gegebenenfalls vorteilhaft, die erfindungsgemäßen Wirkstoffkombinationen mit anderen Wirkstoffen zu kombinieren, beispielsweise mit antimikrobiell wirksamen Stoffen.

Es ist vorteilhaft, die erfindungsgemäßen Zusammensetzungen abzupuffern. Vorteilhaft ist ein pH-Bereich von 3,5 - 7,5. Besonders günstig ist es, den pH-Wert in einem Bereich von 4,0 - 6,0 zu wählen.

Die erfindungsgemäßen kosmetischen oder dermatologischen Zubereitungen können wie üblich zusammengesetzt sein und zur Behandlung der Haut oder der Haare im Sinne einer dermatologischen Behandlung oder einer Behandlung im Sinne der pflegenden Kosmetik dienen. Sie können aber auch in Schminkprodukten in der dekorativen Kosmetik eingesetzt werden.

Zur Anwendung werden die erfindungsgemäßen kosmetischen und/oder dermatologischen Formulierungen in der für Kosmetika und Dermatika üblichen Weise auf die Haut und/oder die Haare in ausreichender Menge aufgebracht.

Vorteilhaft sind solche kosmetische und dermatologische Zubereitungen, die in der Form eines Sonnenschutzmittels vorliegen. Vorteilhaft enthalten diese zusätzlich mindestens einen UVA-Filter und/oder mindestens einen UVB-Filter und/oder mindestens ein anorganisches Pigment.

Kosmetische Zubereitungen gemäß der Erfindung zum Schutze der Haut vor UV-Strahlen können in verschiedenen Formen vorliegen, wie sie z.B. üblicherweise für diesen Typ von Zubereitungen eingesetzt werden. So können sie z.B. eine Lösung, eine Emulsion vom Typ Wasser-in-Öl (W/O) oder vom Typ Öl-in-Wasser (O/W), oder eine multiple Emulsion, beispielsweise vom Typ Wasser-in-Öl-in-Wasser (W/O/W), ein Gel, eine Hydrodispersion, einen festen Stift oder auch ein Aerosol darstellen.

Die erfindungsgemäßen kosmetischen Zubereitungen können kosmetische Hilfsstoffe enthalten, wie sie üblicherweise in solchen Zubereitungen verwendet werden, z.B. Konservierungsmittel, Bakterizide, Antioxidantien, Parfüme, Mittel zum Verhindern des Schäumens, Farbstoffe, Pigmente, die eine färbende Wirkung haben, Verdickungsmittel, oberflächenaktive Substanzen, Emulgatoren, weichmachende Substanzen, anfeuchtende und/oder feuchthaltende Substanzen, Fette, Öle, Wachse oder andere übliche Bestandteile einer kosmetischen Formulierung wie Alkohole, Polyole, Polymere, Schaumstabilisatoren, Elektrolyte, organische Lösungsmittel oder Silikonderivate.

Sofern die kosmetische oder dermatologische Zubereitung eine Lösung oder Lotion darstellt, können als Lösungsmittel verwendet werden:
- Wasser oder wäßrige Lösungen;
- Öle, wie Triglyceride der Caprin- oder der Caprylsäure, vorzugsweise aber Rizinusöl;
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Alkohole, Diole oder Polyole niedriger C-Zahl, sowie deren Ether, vorzugsweise Ethanol, Isopropanol, Propylenglykol, Glycerin, Ethylenglykol, Ethylenglykolmonöethyl- oder -monobutylether, Propylenglykolmonomethyl, -monoethyl- oder -monobutylether, Diethylenglykolmonomethyl- oder -monoethylether und analoge Produkte.

Insbesondere werden Gemische der vorstehend genannten Lösungsmittel verwendet. Bei alkoholischen Lösungsmitteln kann Wasser ein weiterer Bestandteil sein.

Erfindungsgemäß können als günstige Antioxidantien alle für kosmetische und/oder dermatologische Anwendungen geeigneten oder gebräuchlichen Antioxidantien verwendet werden.

Vorteilhaft werden die Antioxidantien gewählt aus der Gruppe bestehend aus Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ - Linoleyl-, Cholesteryl - und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Zitronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. γ -Linolensäure, Linolsäure, Ölsäure), Folsäure und deren Derivate, Ubichinon und Ubichinol und deren Derivate, Vitamin C und Derivate (z.B. Ascorbylpalmitat, Mg - Ascorbylphosphat, Ascorbylacetat), Tocopherole und Derivate (z.B. Vitamin E - acetat), Vitamin A und Derivate (Vitamin A - palmitat) sowie Konyferylbenzoat des Benzoeharzes, Rutinsäure und deren Derivate, Ferulasäure und deren Derivate, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z.B. ZnO, ZnSO₄ Selen und dessen Derivate (z.B. Selenmethionin), Stilbene und deren Derivate (z.B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Die Menge der Antioxidantien (eine oder mehrere Verbindungen) in den Zubereitungen beträgt vorzugsweise 0,001 bis 30 Gew.-%, besonders bevorzugt 0,05 - 20 Gew.-%, insbesondere 1 - 10 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Sofern Vitamin A, bzw. Vitamin-A-Derivate, bzw. Carotine bzw. deren Derivate das oder die Antioxidantien darstellen, ist vorteilhaft, deren jeweilige Konzentrationen aus dem Bereich von 0,001 - 10 Gew.%, bezogen auf das Gesamtgewicht der Formulierung, zu wählen.

Erfindungsgemäße Emulsionen sind vorteilhaft und enthalten z.B. die genannten Fette, Öle, Wachse und anderen Fettkörper, sowie Wasser und einen Emulgator, wie er üblicherweise für einen solchen Typ der Formulierung verwendet wird.

Gele gemäß der Erfindung enthalten üblicherweise Alkohole niedriger C-Zahl, z.B. Ethanol, Isopropanol, 1,2-Propandiol, Glycerin und Wasser bzw. ein vorstehend genanntes Öl in Gegenwart eines Verdickungsmittels, das bei ölig-alkoholischen Gelen vorzugsweise Siliciumdioxid oder ein Aluminiumsilikat, bei wäßrig-alkoholischen oder alkoholischen Gelen vorzugweise ein Polyacrylat ist.

Feste Stifte gemäß der Erfindung enthalten z.B. natürliche oder synthetische Wachse, Fettalkohole oder Fettsäureester. Bevorzugt werden Lippenpflegestifte sowie desodorierende Stifte ("Deo-Sticks").

Als Treibmittel für erfindungsgemäße, aus Aerosolbehältern versprühbare kosmetische oder dermatologische Zubereitungen sind die üblichen bekannten leichtflüchtigen, verflüssigten Treibmittel, z.B. Kohlenwasserstoffe (Propan, Butan, Isobutan) geeignet, die allein oder in Mischung miteinander eingesetzt werden können. Auch Druckluft ist vorteilhaft zu verwenden.

Natürlich weiß der Fachmann, daß es an sich nichttoxische Treibgase gibt, die grundsätzlich für die vorliegende Erfindung geeignet wären, auf die aber dennoch wegen bedenklicher Wirkung auf die Umwelt oder sonstiger Begleitumstände verzichtet werden sollte, insbesondere Fluorkohlenwasserstoffe und Fluorchlorkohlenwasserstoffe (FCKW).

Bevorzugt können die erfindungsgemäßen Zubereitungen zudem Substanzen enthalten, die UV-Strahlung im UVB-Bereich absorbieren, wobei die Gesamtmenge der Filtersubstanzen z.B. 0,1 Gew.-% bis 30 Gew.-%, vorzugsweise 0,5 bis 10 Gew.-%, insbesondere 1 bis 6 Gew.-% beträgt, bezogen auf das Gesamtgewicht der Zubereitung, um kosmetische Zubereitungen zur Verfügung zu stellen, die die Haut vor dem gesamten Bereich der ultravioletten Strahlung schützen. Sie können auch als Sonnenschutzmittel dienen.

Kosmetische Zubereitungen gemäß der vorliegenden Erfindung können auch anorganische Pigmente enthalten, die üblicherweise in der Kosmetik zum Schutze der Haut vor UV-Strahlen verwendet werden. Dabei handelt es sich um Oxide des Titans, Zinks, Eisens, Zirkoniums, Siliciums, Mangans, Aluminiums, Cers und Mischungen davon, sowie Abwandlungen, bei denen die Oxide die aktiven.Agentien sind. Besonders bevorzugt handelt es sich um Pigmente auf der Basis von Titandioxid.

Bei kosmetischen Zubereitungen zur Pflege der Haare handelt es sich beispielsweise um Shampoonierungsmittel, Zubereitungen, die beim Spülen der Haare vor oder nach der Shampoonierung, vor oder nach der Dauerwellbehandlung, vor oder nach der Färbung oder Entfärbung der Haare angewendet werden, um Zubereitungen zum Fönen oder Einlegen der Haare, Zubereitungen zum Färben oder Entfärben, um eine Frisier- und Behandlungslotion, einen Haarlack oder um Dauerwellmittel.

Die kosmetischen Zubereitungen enthalten Wirkstoffe und Hilfsstoffe, wie sie üblicherweise für diesen Typ von Zubereitungen zur Haarpflege und Haarbehandlung verwendet werden.

Als Hilfsstoffe dienen Konservierungsmittel, oberflächenaktive Substanzen, Substanzen zum Verhindern des Schäumens, Emulgatoren, Verdickungsmittel, Fette, Öle, Wachse, organische Lösungsmittel, Bakterizide, Parfüme, Farbstoffe oder Pigmente, deren Aufgabe es ist, die Haare oder die Zubereitung selbst zu färben, Elektrolyte, Zubereitungen gegen das Fetten der Haare.

Erfindungsgemäße wäßrige kosmetische Reinigungsmittel oder für die wäßrige Reinigung bestimmte wasserarme oder wasserfreie Reinigungsmittelkonzentrate können anionische, nichtionische und/oder amphotere Tenside enthalten, beispielsweise herkömmliche Seifen, z.B. Fettsäuresalze des Natriums, Alkylsulfate, Alkylethersulfate, Alkan- und Alkylbenzolsulfonate, Sulfoacetate, Sulfobetaine, Sarcosinate, Amidosulfobetaine, Sulfosuccinate, Sulfobernsteinsäurehalbester, Alkylethercarboxylate, Eiweiß-Fettsäure-Kondensate, Alkylbetaine und Amidobetaine, Fettsäurealkanolamide, Polyglycolether-Derivate.

Die haarkosmetischen Reinigungsmittel und Zubereitungen, die erfindungsgemäße Wirkstoffkombinationen enthalten, sind topische Zubereitungen. Diese können wie üblich zusammengesetzt sein und zur Behandlung und der Pflege der Kopfhaut und/oder der Haare dienen. Zur Anwendung werden die erfindungsgemäßen Zubereitungen in der für Kosmetika und Haarreinigungsmittel üblichen Weise auf die Kopfhaut und die Haare in ausreichender Menge aufgebracht.

Vorteilhaft können Zubereitungen im Sinne der vorliegenden Erfindung auch als Emulsionen oder Lösungen vorliegen.

Die Mittel gemäß der Erfindung können beispielsweise als aus Quetschflaschen oder durch eine Pump- oder Sprühvorrichtung dosierbare Präparate vorliegen, jedoch auch insbesondere in Form eines aus normalen Flaschen und Behältern auftragbären Mittels.

Vorteilhaft enthalten erfindungsgemäße Zubereitungen neben einem wirksamen Gehalt an erfindungsgemäßen Wirkstoffkombinationen ferner übliche Wirk-, Inhalts-, Zusatz- und/oder Hilfsstoffe.

Erfindungsgemäße Zubereitungen, die haarkosmetische Reinigungszubereitungen für das Haar bzw. die Kopfhaut darstellen, können in flüssiger oder fester Form vorliegen. Sie enthalten vorzugsweise mindestens eine anionische, nicht-ionische oder amphotere oberflächenaktive Substanz oder Gemische daraus, gegebenenfalls einen Elektrolyten und Hilfsmittel, wie sie üblicherweise dafür verwendet werden. Die oberflächenaktive Substanz kann in einer Konzentration zwischen 1 und 94 Gew.-% in den Reinigungszubereitungen vorliegen, bezogen auf das Gesamtgewicht der Zubereitungen, insbesondere aber zwischen 1 und 50 Gew.-%.

Insbesondere können erfindungsgemäße wäßrige kosmetische Reinigungsmittel oder für die wäßrige Reinigung bestimmte wasserarme oder wasserfreie Reinigungsmittelkonzentrate anionische, nichtionische und/oder weitere amphotere Tenside enthalten, beispielsweise herkömmliche Seifen, z.B. Fettsäuresalze des Natriums, Alkylsulfate, Alkylethersulfate, Alkan- und Alkylbenzolsulfonate, Sulfoacetate, Sulfobetaine, Sarcosinate, Amidosulfobetaine, Sulfosuccinate, Sulfobernsteinsäurehalbester, Alkylethercarboxylate, Eiweiß-Fettsäure-Kondensate, Alkylbetaine und Amidobetaine, Fettsäurealkanolamide, Polyglycolether-Derivate enthalten.

Anionische Tenside werden vorzugsweise in Konzentrationenzwischen 5 Gew.-% und 20 Gew.-% eingesetzt. In Frage kommen z.B. Natriumlaurylethersulfat wie es unter der Bezeichnung *Texapon N* 70 von der Fa. Henkel angeboten wird oder Dinatriumlaurylethersulfosuccinat wie es unter der Bezeichnung *Rewopol SBFA* 30 von der Fa. Witco angeboten wird.

Nichtionische Tenside werden vorzugsweise in Konzentrationen von 1 Gew.-% bis 10 Gew.-% eingesetzt. Beispiele sind Decylglucoside wie es unter der Bezeichnung *Oramix NS 10* von der Fa. Seppic angeboten wird oder Polysorbat 80 wie es unter der Bezeichnung *Tween* 80 von der Fa. ICI angeboten wird.

Amphotere Tenside werden vorzugsweise in Konzentrationen von 1 Gew.-% bis 10 Gew.-% eingesetzt. Beispiele sind Cocamidopropylbetain wie es als *Tego Betain* von der Fa. Goldschmidt angeboten wird oder Natriumcocoamphoacetat wie es unter der Bezeichnung *Miranol Ultra* von der Fa. Rhone Poulenc angeboten wird.

Die Prozentangaben beziehen sich auf das Gesamtgewicht der Zubereitungen.

Weiterhin können in den haarkosmetischen Reinigungsmitteln Konditionierhilfsmittel enthalten sein, z.B. in Mengen von 0,001 bis 10 Gew.-%, bezogen auf das Gesamtgewicht der Zubereitungen. Zu den bevorzugten Konditionierhilfsmitteln gehören polymere quaternäre Verbindungen (Quats). Polymere Quats werden vielfach in Shampoos z.B. mit einer Konzentration von 0,01 bis 2 Gew.-% eingesetzt.

Erfindungsgemäß vorteilhafte Konditionierhilfsmittel können dabei aus den in Tabelle 1 aufgelisteten Verbindungen gewählt werden.

| Tabelle 1: Erfindungsgemäß vorteilhafte Konditionierhilfsmittel | | | |
|---|---|---|---|
| **Bezeichnung nach INCI** | **CAS-Nummer** | **Polymertyp** | **Beispiel (Handelsname**) |
| Polyquaternium-2 | CAS 63451-27-4 | Urea, N, N'- bis[3- (dimethylamino)propyl]-, polymer mit 1, 1'- oxybis(2- chloroethan) | Mirapol® A-15 |
| Polyquaternium-5 | CAS 26006-22-4 | Acrylamid, β-Methacryloxyethyltriethylammoniummethosulfat | |
| Polyquaternium-6 | CAS 26062-79-3 | N,N-Dimethyl-N-2-propenyl-2-propen-aminiumchlorid | Merquat® 100 |
| Polyquaternium-7 | CAS 26590-05-6 | N,N-Dimethyl-N-2-propenyl-2-propen-aminiumchlorid, 2-Propenamid | Merquat® S |
| Polyquaternium-10 | CAS 53568-66-4, CAS 55353-19-0, CAS 54351-50-7, CAS 68610-92-4, CAS 81859-24-7 | Quaternäres Ammoniumsalz der Hydroxyethylcellulose | Ceiquat® SC-230M |
| Polyquaternium-11 | CAS 53633-54-8 | Vinylpyrrolidon/dimethylaminoethylMethacrylat-Copolymer/Diethylsulfat-Reaktionsprodukt | Gafquat®755N |
| Polyquaternium-16 | CAS 29297-55-0 | Vinylpyrrolidon/vinylimidazolinium-methochlorid-Copolymer | Luviquat® HM552 |
| Polyquaternium-17 | CAS 90624-75-2 | | Mirapol®AD-1 |
| Polyquaternium-19 | CAS 110736-85-1 | Quaternisierter wasserlöslicher Polyvinylalkohol | |
| Polyquaternium-20 | CAS 110736-86-2 | In Wasser dispergierbarer quaternisierter Polyvinyloctadecylether | |
| Polyquaternium-21 | | Polysiloxan-polydimethyldimethylammoniumacetat-Copolymer | Abil® B 9905 |
| Pofyquaternium-22 | CAS 53694-17-0 | Dimethyldiallylammoniumchlorid/Acrytsäure-Copolymer . | Merquat®280 |
| Polyquaternium-24 | CAS 107987-23-5 | Polymeres quaternäres Ammoniumsalz der Hydroxyethylcellulose | Quartisoft® LM-200 |
| Polyquaternium-28 | CAS 131954-48-8 | Vinylpyrrolidon/Methacrylamidopropyltrimethylam moniumchlorid-Copolvmer | Gafquat®HS-100 |
| Polyquatemium-29 | CAS 92091-36-6, CAS 148880-30-2 | Chitosan, das mit Propylenoxid umgesetzt u. mit Epichlorhydrin quatemisiert wurde | Lexquat® CH |
| Polyquaternium-31 | CAS 136505-02-7, 139767-67-7 | Polymeres, quaternäres Ammoniumsalz, das durch die Umsetzung von DMAPA-Acrylate/Acrylsäure/ Acrylonitrogens-Copolymeren u. Diethylsulfat hergestellt wird | Hypan® QT 100 |
| Polyquaternium-32 | CAS 35429-19-7 | N,N,N-Trimethyl-2-{[82-methyl-1-oxo-2-propenyl)oxy]-ethanaminiumchlorid, polymer mit 2-Propenamid | |
| Polyquaternium-37 | CAS 26161-33-1 | | |
| Polyquaternium-44 | | Copolymeres quaternes Ammoniumsalz aus Vinylpyrrolidon und quaternisiertem Imidazolin | |

Weitere erfindungsgemäß vorteilhafte Konditionierhilfsmittel stellen Cellulosederivate und quaternisierte Guar Gum Derivate, insbesondere Guar Hydroxypropylammoniumchlorid (z.B. Jaguar Excel®, Jaguar C 162® der Firma Rhodia, CAS 65497-29-2, CAS 39421-75-5) dar.

Ein erfindungsgemäß besonders vorteilhaftes Konditionierhilfsmittel stellt Polyquaternium-10 dar, wie es unter der Bezeichnung Polymer JR 400 von der Fa. Amerchol angeboten wird.

Die erfindungsgemäßen Zubereitungen können kosmetische Hilfsstoffe enthalten, wie sie üblicherweise in solchen Zubereitungen verwendet werden, z.B. Konservierungsmittel, Parfüme, Substanzen zum Verhindern des Schäumens, Schaumstabilisatoren, Farbstoffe, Pigmente, die eine färbende Wirkung haben, Verdickungsmittel, oberflächenaktive Substanzen, Emulgatoren, weichmachende, anfeuchtende und/oder feuchhaltende Substanzen, rückfettende Agentien, Fette, Öle, Wachse, Alkohole, Polyole und deren toxikologisch verträglichen Ether und Ester, verzweigte und/oder unverzweigte Kohlenwasserstoffe, weitere Antioxidantien, Stabilisatoren, pH-Wert-Regulatoren, Konsistenzgeber, Bakterizide, Desodorantien, antimikrobielle Stoffe, Antistatika, UV-Absorber, Komplexierungs- und Sequestrierungsagentien, Perlglanzagentien, Polymere, Elektrolyte, organische Lösungsmittel, Silikonderivate, Pflanzenextrakte, Vitamine und/oder andere Wirkstoffe oder andere übliche Bestandteile einer kosmetischen oder dermatologischen Formulierung. Auch Lösungsvermittler, z.B. zur Einarbeitung hydrophober Komponenten wie z.B. von Parfümzubereitungen können enthalten sein.

Die Gesamtmenge der Hilfsstoffe beträgt beispielsweise 0,001 bis 15 Gew.-%, vorzugsweise. 0,01 bis 10 Gew.-%, jeweils bezogen auf das Gesamtgewicht der Zubereitung.

Der Wassergehalt der Zubereitungen beträgt beispielsweise 50 bis 95 Gew.-%, vorzugsweise 55 bis 90 Gew.-%, jeweils bezogen auf das Gesamtgewicht der Zubereitung.

Liegt die kosmetische oder dermatologische Zubereitung in Form einer Lotion vor, die ausgespült und z.B. vor oder nach der Entfärbung, vor oder nach der Shampoonierung, zwischen zwei Shampoonierungsschritten, vor oder nach der Dauerwellbehandlung angewendet wird, so handelt es sich dabei z.B. um wäßrige oder wäßrig-alkoholische Lösungen, die gegebenenfalls oberflächenaktive Substanzen enthalten, bevorzugt nichtionische oder kationische oberflächenaktive Substanzen, deren Konzentration zwischen 0,1 und 10 Gew.-%, vorzugsweise zwischen 0,2 und 5 Gew.-%, liegen kann. Diese kosmetische oder dermatologische Zubereitung kann auch ein Aerosol mit den üblicherweise dafür verwendeten Hilfsmitteln darstellen.

Eine kosmetische Zubereitung in Form einer Lotion, die nicht ausgespült wird, insbesondere eine Lotion zum Einlegen der Haare, eine Lotion, die beim Fönen der Haare verwendet wird, eine Frisier- und Behandlungslotion, stellt im allgemeinen eine wäßrige, alkoholische oder wäßrig-alkoholische Lösung dar und enthält mindestens ein kationisches, anionisches, nichtionisches oder amphoteres Polymer oder auch Gemische davon sowie die erfindungsgemäßen Wirkstoffkombinationen. Die Menge der verwendeten Hydrochinonderivate liegt z.B. zwischen 0,1 und 10 Gew.-%, bevorzugt zwischen 0,1 und 3 Gew.-%.

Kosmetische und dermatologische Zubereitungen zur Behandlung und Pflege der Haare, die die erfindungsgemäßen Wirkstoffkombinationen enthalten, können als Emulsionen vorliegen, die vom nicht-ionischen oder anionischen Typ sind. Nicht-ionische Emulsionen enthalten neben Wasser Öle oder Fettalkohole, die beispielsweise polyethoxyliert oder polypropoxyliert sein können, oder auch Gemische aus den beiden organischen Komponenten. Diese Emulsionen enthalten gegebenenfalls kationische oberflächenaktive Substanzen. Anionische Emulsionen sind vorzugsweise vom Typ einer Seife und enthalten mindestens eine erfindungsgemäße ethoxylierte oder propoxylierte organische Verbindung mit anionischem oder nicht-ionischem Charakter.

Kosmetische und dermatologische Zubereitungen zur Behandlung und Pflege der Haare können als Gele vorliegen, die neben den erfindungsgemäßen Wirkstoffkombinationen und dafür üblicherweise verwendeten Lösungsmitteln noch organische Verdickungsmittel, z.B. ethoxylierte und hydrogenierte. Glycerylfettsäureester, Gummiarabikum, Xanthangummi, Natriumalginat, Cellulose-Derivate, vorzugsweise Methylcellulose, Hydroxymethylcellulose, Hydroxyethylcellulose, Hydroxypropylcellulose, Hydroxypropylmethylcellulose oder anorganische Verdickungsmittel, z.B. Aluminiumsilikate wie beispielsweise Bentonite, oder ein Gemisch aus Polyethylenglykol und Polyethylenglykolstearat oder -distearat, enthalten.

Ferner ist es erfindungsgemäß von Vorteil, wenn in den erfindungsgemäßen Zubereitungen Salze eingesetzt werden. Diese führen zu einer Verdickung der Zubereitung, so daß Zubereitungen mit Viskositäten von 2000 mPas bis 6000 mPas hergestellt werden können. Unter Salzen sind ein- oder mehrwerteige Alkalimetallverbindungen bzw. Erdalkalimetallverbindungen mit Halogenanionen zu verstehen. Besonders bevorzugt wird Natriumchlorid eingesetzt.

Das Verdickungsmittel ist im Gel z.B. in einer Menge zwischen 0,1 und 30 Gew.-%, bevorzugt zwischen 0,5 und 15 Gew.-%, enthalten.

Vorteilhafte Ausführungsformen der erfindungsgemäßen kosmetischen Zubereitung sind auch dadurch gekennzeichnet, daß sie als weitere Bestandteile. Trübungsmittel und/ oder Perlglanzmittel enthalten. Als Trübungsmittel werden dabei erfindungsgemäß Substanzen und Substanzgemische verstanden, welche der Zubereitung ein trübes emulsionsartiges Aussehen verleihen.

Als Perlglanzmittel werden dabei erfindungsgemäß Substanzen und Substanzgemische verstanden, welche der Zubereitung ein opaleszierendes Aussehen verleihen.

Erfindungsgemäß vorteilhaft.ist es auch Mischungen aus Trübungs- und Perlglanzmittel einzusetzen.

Erfindungsgemäß vorteilhafte Trübungsmittel/Perlglanzmittel bzw. Mischungen sind unter anderem:
▪ PEG-3 Distearat (z.B. CUTINA TS der Firma Cognis),
▪ eine Kombination aus Glycoldistearat, Glycerin, Laureth-4 und Cocamidopropylbetain (z.B. Euperlan PK 3000 und Euperlan PK 4000 der Firma Cognis),
▪ eine Kombination aus Glycoldistearat, Cocosglucosiden, Glyceryloleat und Glycerylstearat (z.B. Lamesoft TM Benz der Firma Cognis).
▪ Styrol/Acrylat Copolymere (z.B. Acusol OP 301 von Rohm & Haas)

Die folgenden Beispiele sollen die Verkörperungen der vorliegenden Erfindungen verdeutlichen. Die Angaben beziehen sich stets auf Gewichts-%, sofern nicht andere Angaben gemacht werden.

### Beispiele 1 - 3

| Conditioner-Shampoo mit Perlglanz | | | |
|---|---|---|---|
| | **1** | **2** | **3** |
| Polyquaternium-10 | 0,5 | 0,5 | 0,5 |
| Natriumlaurethsulfat | 9,0 | 9,0 | 9,0 |
| Undecylenamidopropylbetain | 2,5 | 3,5 | 2,0 |
| Perlglanzmittel | 2,0 | 2,0 | 2,0 |
| Climbazol | 1,5 | 1,0 | 0,5 |
| Pirocton olamin | 0,3 | 0,5 | 0,8 |
| Laureth-9 Konservierungsmittel, Parfüm, Verdicker, pH-Einstellung und | 0,5 | 2,0 | 1,5 |
| Lösungsvermittler | q.s. | q.s. | q.s. |
| Wasser, VES (vollentsalzt) | ad 100,0 | ad 100,0 | ad 100,0 |

Der pH-Wert wird auf 6 eingestellt.

### Beispiele 4 - 6

| klares Conditioner-Shampoo | | | |
|---|---|---|---|
| | **4** | **5** | **6** |
| Polyquaternium-10 | 0,5 | 0,5 | 0,5 |
| Natriumlaurethsulfat | 9,0 | 9,0 | 9,0 |
| Undecylenamidopropylbetain | 3,5 | 2,5 | 4,5 |
| Climbazole | 0,7 | 0,3 | 0,5 |
| Pirocton olamin | 0,1 | 0,2 | 0,3 |
| Laureth-9 Konservierungsmittel, Parfüm, Verdicker, pH-Einstellung und | 2,5 | 1,3 | 0,7 |
| Lösungsvermittler | q.s. | q.s. | q.s. |
| Wasser, VES | ad 100,0 | ad 100,0 | ad 100,0 |

Der pH-Wert wird auf 6 eingestellt.

### Beispiele 7 - 9

| klares Light-Shampoo mit Volumeneffekt | | | |
|---|---|---|---|
| | **7** | **8** | **9** |
| Natriumlaurethsulfat | 10,0 | 10,0 | 10,0 |
| Undecylenamidopropylbetain | 1,0 | 2,0 | 2,5 |
| Climbazol | 0,3 | 0,4 | 2,0 |
| Pirocton olamin | 0,2 | 0,3 | 0,8 |
| Laureth-9 Konservierungsmittel, Parfüm, Verdicker, pH-Einstellung und | 0,2 | 0,7 | 1,5 |
| Lösungsvermittler | q.s. | q.s. | q.s. |
| Wasser, VES | ad 100,0 | ad 100,0 | ad 100,0 |

Der pH-Wert wird auf 5,5 eingestellt.

## Patentansprüche

1. Wirkstoffkombinationen, umfassend
a) Climbazol
b) Pirocton olamin
c) Undecylenamidopropylbetain,

2. Wirkstoffkombinationen nach Anspruch 1, **dadurch gekennzeichnet, daß** die Gewichtsverhältnisse von Climbazol zu Pirocton olamin zu Undecylenamidopropylbetain wie a : b : c gewählt werden, wobei a, b und c unabhängig voneinander rationale Zahlen von 1 bis 10, bevorzugt von 1 bis 8 darstellen können. Insbesondere bevorzugt ist ein Gewichtsverhältnis von etwa 2:1:8.

3. Kosmetische oder dermatologische Zubereitungen mit einem Gehalt an Wirkstoffkombinationen gemäß Anspruch 1 oder 2.

4. Zubereitungen nach Anspruch 3, **dadurch gekennzeichnet, daß** sie 0,001 bis 5,0 Gew.-% Climbazol enthalten, bevorzugt 0,01 bis 2,0 Gew.-%, bezogen auf das Gesamtgewicht der Zubereitungen.

5. Zubereitungen nach Anspruch 3 oder 4, **dadurch gekennzeichnet, daß** sie 0,001 bis 5,0 Gew.-% Pirocton olamin enthalten, bevorzugt 0,01 bis 2,0 Gew.-%, jeweils bezogen auf das Gesamtgewicht der Zubereitungen.

6. Zubereitungen nach einem der vorhergehenden Ansprüche, **dadurch gekennzeichnet, daß** sie 0,01 bis 25 Gew.-% Undecylenamidopropylbetain enthalten, bevorzugt 0,02 bis 5 Gew.-%, jeweils bezogen auf das Gesamtgewicht der Zubereitungen.

7. Zubereitungen nach einem der vorhergehenden Ansprüche, **dadurch gekennzeichnet, daß** sie zusätzlich 0,1 - 10,0 Gew.-% eines Alkylpolyglykolethers, enthalten.

8. Zubereitungen nach einem der vorhergehenden Ansprüche, **dadurch gekennzeichnet, daß** sie Laureth-9 enthalten, in Konzentrationen von 0,5 - 7,0 Gew.-% jeweils bezogen auf das Gesamtgewicht der Zubereitung.

9. Wirkstoffkombinationen nach einem der vorhergehenden Ansprüche zur Verwendung als Mittel zur Bekämpfung oder Prophylaxe von Mycobionten, insbesondere Pityrosporum ovale..

10. Wirkstoffkombinationen nach einem der vorhergehenden Ansprüche zur Verwendung als Mittel zur Bekämpfung oder Prophylaxe von Kopfschuppen.

## Claims

1. Active ingredient combinations comprising
a) climbazole
b) piroctone olamine
c) undecylenamidopropylbetaine.

2. Active ingredient combinations according to Claim 1, **characterized in that** the weight ratios of climbazole to piroctone olamine to undecylenamidopropylbetaine are selected as a:b:c, where a, b and c, independently of one another, can be rational numbers from 1 to 10, preferably from 1 to 8. Particular preference is given to a weight ratio of about 2:1:8.

3. Cosmetic or dermatological preparations with a content of active ingredient combinations according to Claim 1 or 2.

4. Preparations according to Claim 3, **characterized in that** they comprise 0.001 to 5.0% by weight of climbazole, preferably 0.01 to 2.0% by weight, based on the total weight of the preparations.

5. Preparations according to Claim 3 or 4, **characterized in that** they comprise 0.001 to 5.0% by weight of piroctone olamine, preferably 0.01 to 2.0% by weight, in each case based on the total weight of the preparations.

6. Preparations according to one of the preceding claims, **characterized in that** they comprise 0.01 to 25% by weight of undecylenamidopropylbetaine, preferably 0.02 to 5% by weight, in each case based on the total weight of the preparations.

7. Preparations according to one of the preceding claims, **characterized in that** they additionally comprise 0.1-10.0% by weight of an alkylpolyglycol ether,

8. Preparations according to one of the preceding claims, **characterized in that** they comprise laureth-9 in concentrations of 0.5-7.0% by weight, in each case based on the total weight of the preparation.

9. Active ingredient combinations according to one of the preceding claims for use as an agent for the control or prophylaxis of mycobionts, in particular Pityrosporum ovale.

10. Active ingredient combinations according to one of the preceding claims for use as an agent for the control or prophylaxis of dandruff.

## Revendications

1. Combinaisons de substances actives, comprenant :
a) du climbazole,
b) de la piroctone olamine,
c) de l'undécylène-amidopropylbétaïne.

2. Combinaisons de substances actives selon la revendication 1, **caractérisées en ce que** le rapport en poids entre le climbazole et la piroctone olamine et l'undécylène-amido-propylbétaïne est choisi sous la forme a:b:c, a, b et c pouvant représenter indépendamment les uns des autres des nombres rationnels de 1 à 10, de préférence de 1 à 8. Un rapport en poids d'environ 2:1:8 est particulièrement préféré.

3. Préparations cosmétiques ou dermatologiques ayant une teneur en combinaisons de substances actives selon la revendication 1 ou 2.

4. Préparations selon la revendication 3, **caractérisées en ce qu'**elles contiennent 0,001 à 5,0 % en poids de climbazole, de préférence 0,01 à 2,0 % en poids, par rapport au poids total des préparations.

5. Préparations selon la revendication 3 ou 4, **caractérisées en ce qu'**elles contiennent 0,001 à 5,0 % en poids de piroctone olamine, de préférence 0,01 à 2,0 % en poids, à chaque fois par rapport au poids total des préparations.

6. Préparations selon l'une quelconque des revendications précédentes, **caractérisées en ce qu'**elles contiennent 0,01 à 25 % en poids d'undécylène-arnidopropylbétaïne, de préférence 0,02 à 5 % en poids, à chaque fois par rapport au poids total des préparations.

7. Préparations selon l'une quelconque des revendications précédentes, **caractérisées en ce qu'**elles contiennent en outre 0,1 à 10,0 % en poids d'un éther d'alkylpolyglycol.

8. Préparations selon l'une quelconque des revendications précédentes, **caractérisées en ce qu'**elles contiennent du laureth-9, en concentrations de 0,5 à 7,0 % en poids, à chaque fois par rapport au poids total de la préparation.

9. Combinaisons de substances actives selon l'une quelconque des revendications précédentes, destinées à une utilisation en tant qu'agent de lutte ou de prophylaxie contre les mycobiontes, notamment Pityrosporum- ovale.

10. Combinaisons de substances actives selon l'une quelconque des revendications précédentes, destinées à une utilisation en tant qu'agent de lutte ou de prophylaxie contre les pellicules.
